(19) Europäisches Patentamt
European Patent Office
Office européen des brevets

(11) EP 1 876 970 B1

(12) EUROPÄISCHE PATENTSCHRIFT

(45) Veröffentlichungstag und Bekanntmachung des Hinweises auf die Patenterteilung:
**18.01.2017** Patentblatt **2017/03**

(51) Int Cl.:
**A61B 17/12** (2006.01)

(21) Anmeldenummer: **06742747.6**

(22) Anmeldetag: **28.04.2006**

(86) Internationale Anmeldenummer:
**PCT/EP2006/004000**

(87) Internationale Veröffentlichungsnummer:
**WO 2006/114325 (02.11.2006 Gazette 2006/44)**

(54) **VORRICHTUNG ZUR IMPLANTATION VON OKKLUSIONSWENDELN MIT INNENLIEGENDEM SICHERUNGSMITTEL**

DEVICE FOR IMPLANTING OCCLUSION SPIRALS COMPRISING AN INTERIOR SECURING ELEMENT

DISPOSITIF POUR L'IMPLANTATION DE SPIRALES OCCLUSIVES A ELEMENT DE MAINTIEN INTERIEUR

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **28.04.2005 DE 102005019782**

(43) Veröffentlichungstag der Anmeldung:
**16.01.2008** Patentblatt **2008/03**

(73) Patentinhaber: **DENDRON GmbH**
**44801 Bochum (DE)**

(72) Erfinder:
• **MONSTADT, Hermann**
**44797 Bochum (DE)**
• **BODENBURG, Ralph**
**44801 Bochum (DE)**

(74) Vertreter: **Schneiders, Josef**
**Schneiders & Behrendt**
**Rechts- und Patentanwälte**
**Huestrasse 23**
**44787 Bochum (DE)**

(56) Entgegenhaltungen:
EP-A- 0 792 623     WO-A-2004/014239
WO-A1-2004/062509     DE-A1- 10 010 840
US-A1- 2004 002 733     US-B1- 6 468 266

**Beschreibung**

Die Erfindung betrifft eine Vorrichtung zur Implantation von Okklusionswendeln in Körperhohlräumen oder Blutgefäßen, insbesondere Aneurysmen, mit mindestens einer, aus einer Vielzahl von Windungen bestehenden, in einem Katheter in Längsrichtung vorschiebbaren Okklusionswendel und einem das Lumen der Okklusionswendel zumindest teilweise durchlaufenden Sicherungsmittel, wobei das Sicherungsmittel in seinen Endbereichen in der Okklusionswendel festgelegt ist.

Der Einsatz endovaskulärer Techniken zur Okklusion von Körperhohlräumen oder Gefäßen wie Arterien, Venen, Eileitern oder vaskulären Fehlbildungen (z. B. vaskuläre Aneurysmen) ist bekannter Stand der Technik. Die Okklusionswendel wird dabei in der Regel mit Hilfe eines endovaskulären Führungsdrahtes durch einen Katheter in den zu okkludierenden Hohlraum eingeführt und deponiert.

Im Vorfeld der Deponierung werden die zu implantierenden Okklusionswendeln mit Hilfe des Katheters durch das Blutgefäßsystem gesteuert und am Zielort aus dem Katheter in den zu okkludierenden Hohlraum vorgeschoben. Im Idealfalle schließt sich die Abtrennung der Wendel an. Im Falle fehlerhafter Positionierung oder einer für den zu okkludierenden Bereich zu groß dimensionierten Okklusionswendel muß diese jedoch repositioniert oder vollständig in den Katheter zurückgezogen werden, um anschließend eine korrekte Positionierung oder die Einbringung einer korrekt dimensionierten Okklusionswendel zu ermöglichen. Derartige Manöver im Gefäßsystem bergen die Gefahr, dass Teile der Wendel durch Zug- oder Torsionsbeanspruchung auseinander gezogen und so irreversibel elastisch deformiert werden, reißen oder brechen, was eine lebensgefährliche Embolie nach sich ziehen kann.

Um diese Gefahren zu minimieren, ist es u.a. aus der Schrift WO 99/09894 A1 bekannt, ein flexibles Sicherungsmittel in der Okklusionswendel zu befestigen, welches aus einem Polymermaterial besteht. Mit Hilfe eines solchen Sicherungsmittels wird sichergestellt, dass das Zurückziehen der fehlerhaft positionierten Okklusionswendel ohne die Gefahr des Auseinanderziehens von Teilen der Okklusionswendel möglich ist, um auf diese Weise die oben geschilderten Gefahren zu minimieren.

Neben derartigen polymeren Sicherungsmitteln sind auch solche Sicherungsmittel bekannt, die aus einem Metall mit Formgedächtniseigenschaften, beispielsweise Nitinol, gefertigt sind. Derartige Sicherungsmittel sind in der WO 2004/014239 A1 offenbart, das als nächstliegender Stand der Technik angesehen wird.

Die Verwendung von Formgedächtnismetallen weist insbesondere hinsichtlich der Zugfestigkeit im Vergleich zur Verwendung von Polymermaterialien erhebliche Vorteile auf, da selbst bei starker Zugbelastung ein Zerreißen des Sicherungsmittels nicht zu erwarten ist.

Auf der anderen Seite bewirkt die Verwendung eines metallischen Sicherungsmittels, dass die durch den Katheter vorzuschiebende Okklusionswendel verhältnismäßig steif wird, was mit der relativ hohen Biegefestigkeit des als Sicherungsmittel verwendeten Drahtes zusammenhängt. Eine zu steife Ausbildung der Okklusionswendel erschwert jedoch sowohl das Vorschieben der Okklusionswendel durch enge Blutgefäße als auch die Anpassung an den Innenraum eines Aneurysmas, in das die Okklusionswendel eingeführt wird. Da auf der einen Seite eine hohe Flexibilität und auf der anderen Seite eine hohe Zugfestigkeit des Sicherungsmittels gewünscht ist, mussten bislang stets Kompromisse eingegangen werden. Während die Verwendung eines Polymers zwar eine hohe Flexibilität, jedoch häufig keine ausreichende Zugfestigkeit gewährleistete, verhielt es sich bei Verwendung eines Metalldrahtes genau umgekehrt. Ebenso verhält es sich bei der Wahl der Dimension des Sicherungsmittels; ein Draht mit großem Durchmesser gewährleistet zwar eine ausreichend hohe Zugfestigkeit, weist jedoch Nachteile hinsichtlich der Flexibilität auf, während ein dünnerer Draht zwar ausreichend flexibel, nicht jedoch ausreichend zugfest ist. Gemäß der WO 2004/014239 A1 wurden daher bislang vorzugsweise Drähte mit einem Durchmesser zwischen 0,03 bis 0,1 mm verwendet.

EP-A-1 582 152 und WO-A-2004 062 509 offenbaren Okklusionswendeln mit einem Sicherungsmittel, das aus mehreren geflochtenen Drähten besteht, die einen Durchmesser von weniger als 0,02 mm aufweisen.

Ausgehend vom beschriebenen Stand der Technik stellt sich daher die Aufgabe, eine Vorrichtung der eingangs genannten Art mit einem Sicherungsmittel bereitzustellen, wobei das Sicherungsmittel sowohl eine ausreichende Flexibilität als auch eine ausreichende Zugfestigkeit aufweist.

Diese Aufgabe wird erfindungsgemäß gelöst durch eine Vorrichtung zur Implantation von Okklusionswendeln in Körperhohlräumen oder Blutgefäßen, insbesondere Aneurysmen, mit mindestens einer aus einer Vielzahl von Windungen bestehenden, in einem Katheter in Längsrichtung vorschiebbaren Okklusionswendel und einem das Lumen der Okklusionswendel zumindest teilweise durchlaufenden Sicherungsmittel, wobei das Sicherungsmittel in seinen Endbereichen in der Okklusionswendel festgelegt ist und aus mindestens zwei Drähten besteht, wobei jeder Draht einen Durchmesser von weniger als 0,02 mm aufweist.

Der Erfindung liegt die Erkenntnis zugrunde, dass auf der einen Seite die Zugfestigkeit proportional zur Querschnittsfläche der als Sicherungsmittel verwendeten Drähte ansteigt, die Flexibilität des Sicherungsmittel im Ganzen mit der Abnahme des Durchmessers der Drähte jedoch stark überproportional zunimmt. Entsprechend weist etwa ein Sicherungsmittel aus zwei Drähten mit einer bestimmten Querschnittsfläche zwar die gleiche Zugfestigkeit wie ein Sicherungsmittel aus einem Draht mit derselben Querschnittsfläche auf, die Flexibilität des Sicherungsmittel ist jedoch deutlich erhöht.

Theoretisch läßt sich das Phänomen folgendermaßen beschreiben:

Die Berechnung der möglichen Durchbiegung f ergibt sich nach folgender Formel:

$$f = \frac{\frac{F}{n} * l^3}{3 * E * I_D}$$

Hierin bedeuten:

F = Kraft
E = Elastizitätsmodul
n = Anzahl der Drähte
l = Länge
$I_D$ = Axiales Widerstandsmoment,

wobei sich das axiale Widerstandsmoment $I_D$ nach der Formel

$$I_D = \frac{\pi * D^4}{64}$$

berechnet, wobei D der Durchmesser des oder der für das Sicherungsmittel verwendeten Drähte ist. Wie man unschwer erkennt, geht dabei der Durchmesser mit der vierten Potenz in die berechnete Durchbiegung f ein, die zustande kommt, wenn man auf einen Abschnitt des Sicherungsmittel eine bestimmte Kraft F einwirken läßt. Die Durchbiegung f ist somit ein Maß für die Flexibilität des Sicherungsmittels. Die Zahl der verwendeten Drähte n hingegen geht lediglich einfach in die Durchbiegung f ein, so dass mehrere dünne Drähte deutlich flexibler sein können, als ein dickerer Draht.

Die Zugfestigkeit des Sicherungsmittels ist, wie oben bereits erwähnt, direkt proportional zur Gesamtquerschnittsfläche A, wobei sich A aus

$$A = \frac{n * \pi * D^2}{4}$$

ergibt. Der Durchmesser der einzelnen Drähte geht hier lediglich quadratisch ein.

Eine Gegenüberstellung verschiedener, errechneter Flexibilitäten und Zugfestigkeiten ergibt sich aus der nachfolgenden Tabelle:

| NiTi-Draht | 1 x Ø 0,024 mm | 2 x Ø 0,018 mm | 3 x Ø 0,018 mm | 3 x Ø 0,015 mm |
|---|---|---|---|---|
| Durchbiegung (Flexibilität) | 100% | 158 % | 104 % | 218 % |
| Zugfestigkeit | 100% | 112% | 169 % | 117 % |

Wie man erkennt, ist es möglich, etwa durch Verwendung von drei Drähten mit einem Durchmesser von jeweils 0,015 mm anstelle eines Drahtes mit einem Durchmesser von 0,024 mm die Flexibilität um mehr als das Doppelte zu steigern und gleichzeitig sogar die Zugfestigkeit geringfügig zu erhöhen, was bei den aus dem Stand der Technik bekannten Sicherungsmitteln als sich gegenseitig ausschließende Effekte hätte angesehen werden müssen. Gleichzeitig können Drähte für die Herstellung des Sicherungsmittels verwendet werden, die aufgrund ihres geringen Durchmessers wegen zu geringer Zugfestigkeit nicht in Betracht gezogen worden wären.

Erfindungsgemäß werden vorzugsweise zwei bis vier Drähte für das Sicherungsmittel verwendet, wobei sich die Verwendung von drei Drähten gemäß oben gezeigter Tabelle als besonders vorteilhaft herausstellt. Grundsätzlich ist zwar auch die Verwendung von mehr als vier noch dünneren Drähten möglich, die Erhöhung der Zahl der Drähte bei gleichzeitiger Verringerung des Durchmessers der Drähte macht jedoch die Fertigung aufwendiger, so dass sich die Verwendung von zwei bis vier Drähten als vorteilhafter Kompromiss herausgestellt hat.

Die Drähte sind aus einem Metall mit Formgedächtniseigenschaften gefertigt.

Bei den Formgedächtniseigenschaften des Sicherungsmittels kann es sich sowohl um einen thermischen als auch um einen mechanischen Formgedächtniseffekt handeln. Besonders bewährt hat sich hier die Verwendung von Nickel-Titan-Legierungen, insbesondere eine dem Fachmann unter dem Namen Nitinol bekannte Legierung. Darüber hinaus sind jedoch auch Alternativen, z. B. die Verwendung von Eisenbasis- oder Kupferbasislegierungen, möglich. Die genaue Eigenschaft eines Formgedächtnismaterials können in für den Fachmann bekannter Weise durch die genaue Wahl der Zusammensetzung gesteuert werden.

Insbesondere können die Drähte für das Sicherungsmittel auch aus unterschiedlichen Materialien bestehen, um auf diese Art und Weise unterschiedliche Eigenschaften miteinander zu kombinieren. Ferner können die Drähte des Sicherungsmittels durch unterschiedliche Behandlung mit unterschiedlichen Eigenschaften versehen werden, beispielsweise besonders hohe Flexibilität einerseits und besonders hohe Zugfestigkeit andererseits. Ferner ist es möglich, einen oder mehrere der Drähte aus Materialien mit Formgedächtniseigenschaften zu fertigen und einen oder mehrere Drähte aus anderen Materialien, die andere Eigenschaften einbringen.

Darüber hinaus kann das aus einem Formgedächtnismetall hergestellte Sicherungsmittel zu einer übergeordneten Struktur vorgeformt sein, die es beim Ausbringen aus einem zur Platzierung der Okklusionswendel verwendeten Katheter einnimmt. Auf diese Weise kann der Okklusionswendel nach Ausbringung aus dem Katheter etwa in ein Aneurysma eine gewünschte übergeordnete Struktur aufgeprägt werden. Die Ausbildung einer solchen Sekundärstruktur, beispielsweise helikaler Windungen oder einer korbartigen Form, ist grundsätzlich vorteilhaft, da auf diese Weise das Aneurysma besonders effektiv ausgefüllt wird, um eine wirkungsvolle Thrombosierung des Aneurysmas zu gewährleisten. Gegebenenfalls kann hierzu auch die Okklusionswendel selbst zu einer übergeordneten Struktur vorgeformt sein, welche sie nach der Ausbringung aus dem Katheter annimmt. Unter Umständen ist es jedoch ausreichend, wenn lediglich das Sicherungsmittel, nicht aber die Okklusionswendel selbst vorgeformt ist, wenn die vom Sicherungsmittel ausgehende Kraft groß genug ist, die Okklusionswendel ebenfalls in die durch das Sicherungsmittel vorgegebene Form zu zwingen. Die durch das Sicherungsmittel ausgeübte Kraft wird dadurch hervorgerufen, dass das Sicherungsmittel bei Ausbringung in das Aneurysma von dem durch den Katheter ausgeübten Zwang befreit wird und eine Rückumwandlung in seine Austenit-Phase eingeht, wobei es die ihm zuvor aufgeprägte Struktur einnimmt. Zusätzlich oder alternativ kann auch eine temperaturinduzierte Transformation auftreten, wenn das Sicherungsmittel beim Ausschieben aus dem Katheter einer erhöhten Temperatur im Blutstrom ausgesetzt wird.

Die das Sicherungsmittel bildenden Drähte verlaufen erfindungsgemäß parallel zueinander, da in diesem Fall der oben beschriebene vorteilhafte Effekt automatisch auftritt.

Zweckmäßigerweise weist die Okklusionswendel ein oder mehrere elektrolytisch korrodierbare Stellen auf. Die elektrolytische Ablösung von Okklusionswendeln ist dem zuständigen Fachmann hinlänglich bekannt und weist gegenüber anderen, im Stand der Technik bekannten Maßnahmen zur Ablösung von Okklusionswendeln vielfache Vorteile hinsichtlich Praktikabilität, Sicherheit, Schnelligkeit und Kostengünstigkeit auf. Bei der elektrolytischen Ablösung wird ein elektrisch isolierender Katheter und eine Spannungsquelle verwendet, wobei die Okklusionswendel selbst als Anode dient. Eine Kathode wird üblicherweise auf der Körperoberfläche positioniert. Neben dem Vorsehen von als elektrolytisch korrodierbare Stellen dienenden Ablöseelementen in der Okklusionswendel sind aus dem Stand der Technik auch Vorrichtungen bekannt, bei denen die Ablösestelle im Führungsdraht angeordnet ist.

Besonders zweckmäßig ist es, wie aus der DE 100 10 840 A1 bekannt, wenn die Okklusionswendel mehrere elektrolytisch korrodierbare Stellen aufweist, wobei in jedem zwischen diesen Stellen gelegenen Segment der Okklusionswendel ein Sicherungsmittel angeordnet ist. Dieses erstreckt sich vorzugsweise jeweils von einem Ende zum anderen Ende jedes Segmentes. Diese Ausführungsform ermöglicht die Deponierung variabel dimensionierbarer Längen an Okklusionswendeln, so dass die Okklusionswendeln in genau der richtigen Länge im Aneurysma abgesetzt werden können. Gegebenenfalls können auch mehrere Längen derselben Okklusionswendel nacheinander abgelöst und in den zu okkludierenden Hohlraum eingebracht werden. Dies spart nicht nur Kosten und Zeit, sondern dient insbesondere auch der Minimierung des Operationsrisikos. Darüber hinaus wird auf diese Weise gewährleistet, dass für verschieden große Aneurysmen nicht stets unterschiedlich dimensionierte Okklusionswendeln bereitgehalten und verwendet werden müssen, sondern stattdessen eine einheitliche Vorrichtung verwendet werden kann, bei der je nach Bedarf unterschiedlich große Abschnitte der Okklusionswendel in das Aneurysma eingeführt werden können.

Gegebenenfalls können auch mehrere voneinander beabstandete Okklusionswendeln verwendet werden, wobei zwischen den einzelnen Okklusionswendeln jeweils ein elektrolytisch korrodierbar ausgebildetes Ablöseelement angeordnet ist. In diesem Fall sollte in den einzelnen Okklusionswendeln jeweils ein Sicherungsmittel vorgesehen sein.

Die Verwendung von an mehreren Stellen elektrolytisch korrodierbar ausgebildeten Okklusionswendeln beruht auf der Erkenntnis, dass bei Anlegen eines Stroms an eine solche Vorrichtung sich spezifisch die dem distalen Ende des Katheter am nächsten liegende Ablösestelle der Okklusionswendel durch Elektrolyse löst. Dies ist darauf zurückzuführen, dass einerseits die sich im Katheter befindenden elektrolytisch korrodierbaren Stellen durch den Katheter vom ionischen Medium isoliert sind und deshalb keiner Elektrolyse unterliegen können und andererseits die Stromdichte aufgrund des nach distal zunehmenden Widerstandes in der bzw. den Okklusionswendeln von proximal nach distal abnimmt. Die sich nach distal als erstes an das distale Katheterende anschließende elektrolytisch korrodierbar ausgebildete Stelle ist deshalb am stärksten elektrolytischen Prozessen unterworfen und löst sich bevorzugt auf. Das Vorsehen jeweils eines Sicherungsmittels in den Segmenten der Okklusionswendel zwischen den elektrolytisch korrodierbaren Stellen bzw. in den einzelnen Okklusionswendeln dient der Sicherung jedes einzelnen Segmentes und gewährleistet ein Höchstmaß an Sicherheit gegen das Abreißen der Okklusionswendel.

Die das Sicherungsmittel bildenden Drähte können von einer isolierenden Umhüllung umgeben sein. Dies ist insbesondere sinnvoll, wenn eine Vorrichtung mit elektrolytisch korrodierbar ausgebildeten Ablösestellen verwendet wird, weil durch den isolierenden Überzug verhindert wird, dass das Sicherungsmittel selbst durch elektrolytische Korrosion angegriffen wird. Darüber hinaus sorgen Isolierungen dafür, dass die Stromdichte am Ablöseelement möglichst hoch ist, um eine rasche Ablösung zu ermöglichen.

Sinnvollerweise erstreckt sich das Sicherungsmittel vom proximalen bis zum distalen Ende der Okklusionswendel bzw. zumindest des abtrennbaren Segments der Okklusionswendel, um die Sicherung der gesamten Okklusionswendel gegen ein Abreißen zu gewährleisten. Insbesondere ist es auch sinnvoll, wenn sich das Sicherungsmittel bis zum distalen Spitzenabschnitt der Okklusionswendel erstreckt, welcher bei der Einbringung in das Blutgefäß besonders großer Beanspruchung unterliegt und zur Vermeidung von Wandrupturen meist abgerundet ist.

Das Sicherungsmittel kann grundsätzlich an seinen beiden Enden direkt oder mittelbar mit der Okklusionswendel verbunden sein. Bei der mittelbaren Verbindung wird die Festlegung des Sicherungsmittels in der Okklusionswendel durch Übergangsstücke erreicht, die mit dem Sicherungsmittel und der Okklusionswendel verbunden sind. Bei dieser Festlegung kann es sich z. B. um eine Crimp-Verbindung handeln. U. a. können als Übergangsstücke seinerseits Mikrowendeln verwendet werden, die hinsichtlich ihres Außendurchmessers an den Innendurchmesser der Okklusionswendel angepasst und in diese zumindest teilweise eingesetzt sind. Diese Ausführungsform ist besonders kostengünstig, da zur Herstellung herkömmliche Okklusionswendeln verwendet werden können, in welche die Kombination aus Sicherungsmittel und mindestens zwei, an den Enden des Sicherungsmittels befestigten Mikrowendeln eingefügt und über gängige Verfahren mit der Okklusionswendel verbunden werden. Zur Verbindung von Sicherungsmitteln mit Übergangsstück (Mikrowendel) bzw. Übergangsstück mit Okklusionswendel eignen sich dem Fachmann hinlänglich bekannte Maßnahmen wie Verschweißen, Verlöten, Verkleben oder mechanische (d. h. kraft- und/oder formschlüssige) Fügeverfahren. Daneben ist es auch möglich, das Sicherungsmittel zumindest in einem Endbereich über eine reibschlüssige Verbindung an der Okklusionswendel oder dem Übergangsstück festzulegen. Durch die genaue Ausgestaltung der reibschlüssigen Verbindung, die für den Fachmann ohne weiteres möglich ist, lassen sich die Reibungskräfte so einstellen, dass die das Sicherungsmittel festlegenden Reibungskräfte kleiner sind als die Zugkraft, die auf das Sicherungsmittel einwirken muß, um es zum Reißen zu bringen. Auf der anderen Seite muß die Reibungskraft so hoch eingestellt werden, dass im Normalfall das Zurückziehen und Repositionieren der Okklusionswendel problemlos möglich ist. Für den Fall, dass die Zugkräfte so stark ansteigen, dass das Reißen des Sicherungsmittels zu erwarten wäre, zieht sich das Sicherungsmittel an der Stelle seiner Festlegung in der Mikrowendel aus dieser heraus, so dass die reibschlüssige Verbindung gelöst und das Reißen des Sicherungsmittels verhindert wird. Dieser Prozess der Lösung der reibschlüssigen Verbindung erfolgt nicht plötzlich wie das Reißen des Sicherungsmittels sondern sukzessive, so dass keine plötzlich freiwerdenden Kräfte auftreten, die im Blutgefäß negative Auswirkungen zur Folge haben können.

Vorzugsweise ist das Sicherungsmittel der erfindungsgemäßen Vorrichtung etwas länger dimensioniert als der Teilbereich der Okklusionswendel, durch dessen Lumen es sich erstreckt. Diese Längendimensionierung des Sicherungsmittels bewirkt, dass das Sicherungsmittel in der Okklusionswendel ohne äußere Einwirkung keiner Zugbeanspruchung unterliegt und schränkt darüber hinaus die Flexibilität der Okklusionswendel noch weniger ein.

Neben den oben beschriebenen Maßnahmen zur Erhöhung der Flexibilität der Okklusionswendel kann auch für den die Okklusionswendel selbst bildenden Draht ein Draht geringeren Durchmessers als üblich verwendet werden. So ist es möglich, hierfür einen Draht mit einem Durchmesser von lediglich 0,03 mm zu verwenden, was, wie sich gezeigt hat, ebenfalls mit einer Erhöhung der Flexibilität verbunden ist.

Für die Ausbildung der Okklusionswendeln hat sich die Verwendung von Platin oder Platinlegierungen bewährt. Besonders bevorzugt ist dabei die Verwendung von Platin-Iridium-Legierungen. Solche Legierungen weisen eine hohe Röntgendichte auf und machen somit die Visualisierung der Okklusionswendel im Körper möglich.

Die Ablöseelemente, die für eine rasche Korrosion vorgesehen sind, bestehen vorzugsweise aus einer Stahllegierung. Bevorzugt sind hier nichtrostende Stähle mit einem Chromanteil zwischen 12 und 20 Gew.-%. Gegebenenfalls können diese Ablöseelemente z. B. durch Wärmebehandlung vorkorrodiert sein, wodurch das Metall in seinem Gefüge so verändert wird, dass es sich bei Anlegen einer elektrischen Spannung in einem Elektrolyten besonders rasch zersetzt. Eine weitere Möglichkeit, die Ablöseelemente besonders gut korrodierbar auszubilden, besteht darin, in den entsprechenden Bereichen Materialkombinationen auszuwählen, die geeignet sind, Lokalelemente auszubilden. Beispiele hierfür sind Kombinationen aus nichtrostenden Stählen mit Edelmetallen bzw. Edelmetalllegierungen, insbesondere Platinlegierungen.

Zweckmäßigerweise schließt sich proximal an die Okklusionswendel eine als Führungsdraht ausgebildete Einführhilfe an. Derartige Führungsdrähte haben sich bewährt, um Okklusionswendeln durch einen Katheter zum zu okkludierenden Hohlraum zu führen.

Die erfindungsgemäße Vorrichtung kann auch direkt in Kombination mit einem Katheter, insbesondere einem Mikrokatheter, vorliegen, durch den die Okklusionswendel zum zu verschließenden Körperhohlraum oder Blutgefäß vorgeschoben werden kann. Der verwendete Katheter und die verwendete Okklusionswendel sollten dabei hinsichtlich ihrer Dimensionen aufeinander abgestimmt sein. Gegebenenfalls kann der Katheter dabei auch einen Zwang auf die Okklusionswendel sowie das Sicherungsmittel ausüben, der bewirkt, dass die Okklusionswendel erst nach Befreiung von dem Zwang eine ihr bzw. dem Sicherungsmittel zuvor aufgeprägte Sekundärstruktur im Aneurysma einnimmt. Zweckmäßigerweise ist der Katheter darüber hinaus mit röntgendichten Markierungen versehen, die die Platzierung im Zielbereich mit Hilfe bekannter Bildgebungsverfahren ermöglicht.

Die erfindungsgemäße Vorrichtung ist vorzugsweise für den Einsatz in tier- oder humanmedizinischen Verfahren, besonders bei der endovaskulären Behandlung intrakranieller Aneurysmen und erworbener oder angeborener arteriovenöser Gefäßmißbildungen und/oder -fisteln oder der Tumorembolisation durch Thrombosierung bestimmt.

Die Erfindung wird nachfolgend beispielhaft anhand der beigefügten Figuren näher erläutert. Es zeigen:

Figur 1    die schematische Darstellung der Positionierung einer Okklusionswendel in einem Beerenaneurysma mit Hilfe der erfindungsgemäßen Vorrichtung;

Figur 2    den Längsschnitt durch eine erfindungsgemäße Vorrichtung in Seitenansicht und

Figur 3    einen Ausschnitt aus der erfindungsgemäßen Vorrichtung mit Sicherungsmittel in Seitenansicht.

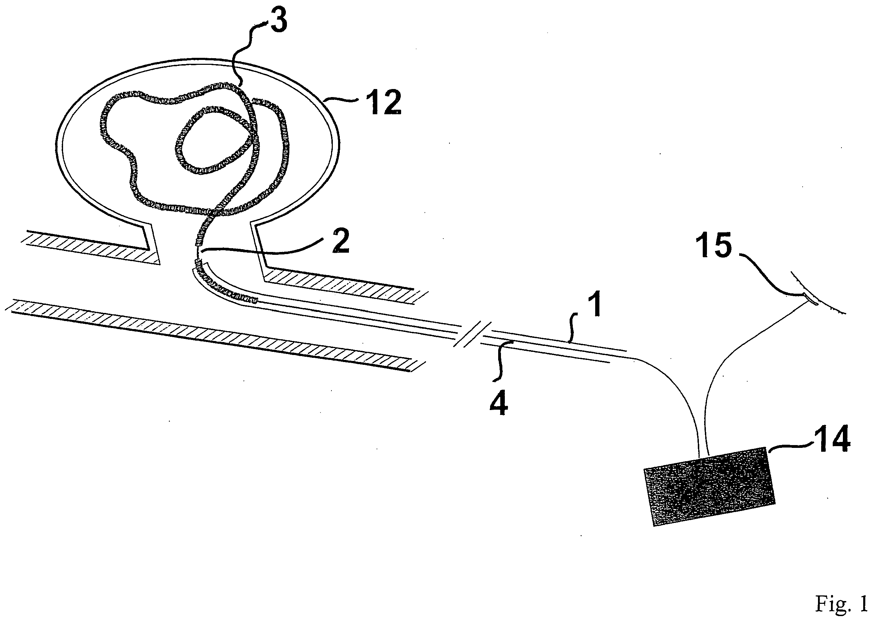

In Figur 1 ist die Ansicht einer in einem Beerenaneurysma 12 positionierten Okklusionswendel 3 dargestellt. Die Okklusionswendel 3 wird mit Hilfe des Führungsdrahtes 4 innerhalb des Katheters 1 nach distal verschoben. Bei korrekter Positionierung tritt die Okklusionswendel 3 aus dem Ende des Katheters 1 heraus in den durch das Beerenaneurysma 12 gebildeten Hohlraum und füllt diesen aus. Innerhalb des Aneurysmas 12 bildet die Okklusionswendel 3 sekundäre Windungen aus, was insbesondere durch einen spannungs- und/oder temperaturinduzierten Übergang der Okklusionswendel 3 und/oder des hier nicht dargestellten Sicherungsmittels im Inneren der Okklusionswendel 3 von der Martensit- in die Austenit-Phase hervorgerufen werden kann. Aufgrund der Ausbildung von sekundären Windungen wird das Aneurysma 12 besonders effektiv ausgefüllt.

Sobald eine an das Volumen des auszufüllenden Hohlraumes angepasste Länge der Okklusionswendel 3 in das Aneurysma 12 eingebracht ist, erfolgt die elektrolytische Ablösung an der elektrolytisch korrodierbaren Stelle 2. Hierzu wird mit Hilfe der Spannungsquelle 14 eine elektrische Spannung an die Stelle 2 angelegt, wobei die elektrolytisch korrodierbare Stelle 2 (Ablöseelement) als Anode dient. Die Kathode 15 wird auf der Körperoberfläche positioniert. Gemäß einer bevorzugten Ausführungsform sind im Bereich der Okklusionswendel 3 mehrere Ablöseelemente 2 vorgesehen, so dass die Länge der eingebrachten Okklusionswendel 3 jeweils individuell an das Aneurysma 12 angepasst werden kann. Die Ablösung erfolgt in der Regel innerhalb kurzer Zeit von 1 min oder weniger (bei 2 V, 2 mA).

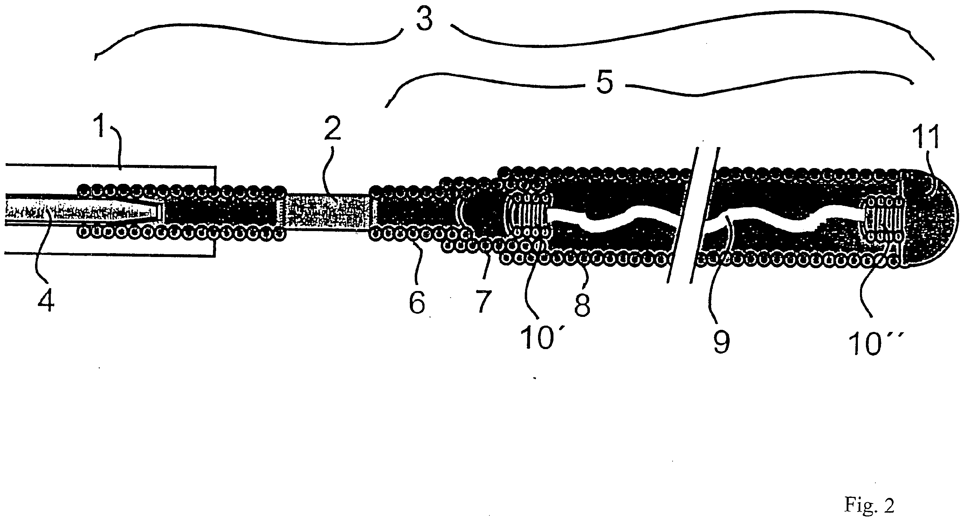

In der Figur 2 ist die erfindungsgemäße Vorrichtung in vergrößerter Darstellung gezeigt. Durch den elektrisch isolierenden Katheter 1, bei dem es sich um einen biegsam ausgebildeten Mikrokatheter handelt, wird die aus einer Platin-IridiumLegierung gefertigte, mit einer elektrolytisch korrodierbaren Stelle 2 aus Edelstahl versehene Okklusionswendel 3 mit Hilfe des schweißtechnisch an die Okklusionswendel 3 gefügten Führungsdrahtes 4 aus dem Mikrokatheter in das Blutgefäßsystem geschoben. Die Okklusionswendel 3 weist ein elektrolytisch abtrennbares Segment 5 auf, das mit der proximal davon angeordneten, elektrolytisch korrodierbaren Stelle 2 durch artfremdes Verschweißen verbunden ist. Das Segment 5 weist an seinem proximalen Ende eine erste Mikrowendel 6 mit geringem Durchmesser auf, die an ihrem proximalen Ende schweißtechnisch mit der sich anschließenden elektrolytisch korrodierbaren Stelle 2 und an ihrem distalen Ende mit einer weiteren Mikrowendel 7 mit mittlerem Durchmesser verbunden ist. Diese Mikrowendel 7 mittleren Durchmessers fügt sich teilweise um die erste Mikrowendel 6 und ist mit dieser ebenfalls schweißtechnisch verbunden. Um die zweite Mikrowendel 7 fügt sich schließlich die am längsten dimensionierte dritte Mikrowendel 8 mit größtem Durchmesser, in deren Innerem das aus einer Nickel-Titan-Legierung bestehende Sicherungsmittel 9 verläuft. Es sei angemerkt, dass es sich bei der hier dargestellten Okklusionswendel 3 lediglich um ein Ausführungsbeispiel handelt und andere Formen der Verbindung von Okklusionswendel 3 und elektrolytisch korrodierbarer Stelle 2 ebenso möglich sind.

Das Sicherungsmittel 9, das, obgleich es aus mehreren Drähten besteht, hier einheitlich dargestellt ist, ist an seinen beiden Enden mit Hilfe von Übergangsstücken 10', 10" in der Okklusionswendel festgelegt. In diesem Fall sind auch die Übergangsstücke 10', 10" wiederum als Mikrowendeln ausgebildet, die jeweils mit den sich anschließenden Mikrowendeln der Okklusionswendel 3 fest verschweißt sind. Die distale Spitze 11 der Okklusionswendel 3 ist abgerundet, um die Gefahr einer Traumatisierung des Aneurysmas oder einer Wandruptur zu minimieren. Im Inneren ist die Spitze 11 mit der distalen, als Verbindungsmittel dienenden Mikrowendel 10" schweißtechnisch fest verbunden, so dass auch im Falle des Abbrechens oder Abreißens der Spitze 11 und angrenzender Bereiche der Okklusionswendel 3 die Spitze 11 nicht in den Blutstrom gelangt und dort Embolien verursachen kann.

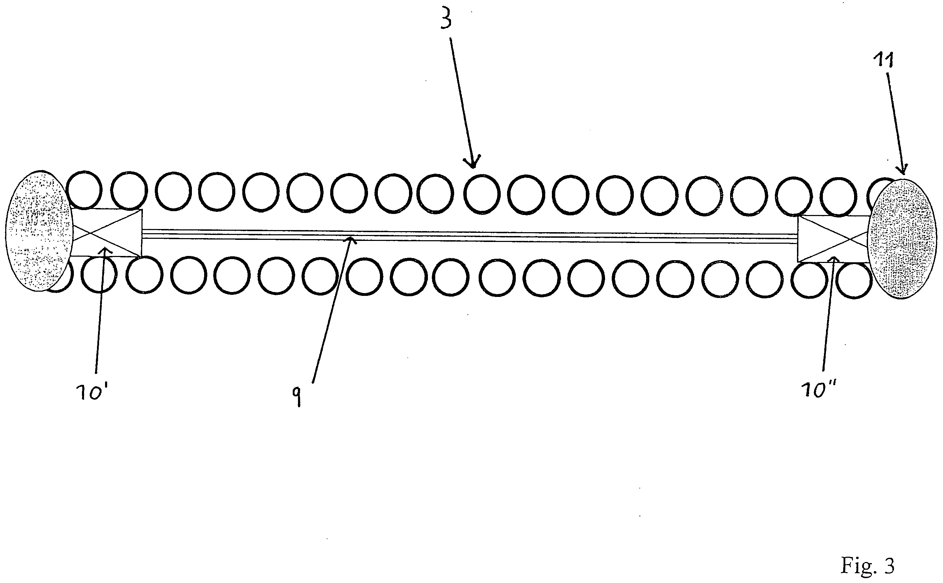

In Figur 3 schließlich ist die Okklusionswendel 3 mit dem Sicherungsmittel 9 gemäß der Erfindung in stark vereinfachter Form dargestellt. Wie man erkennt, besteht das Sicherungsmittel 9 aus insgesamt drei Drähten, die parallel zueinander verlaufen und über Übergangsstücke 10', 10" jeweils mit dem proximalen und distalen Ende der Okklusionswendel 3 verbunden sind, hier jeweils als verpreßte (oder verkrimpte) Mikrowendel ausgeführt. Durch die Verwendung von mehreren Drähten, die dünner ausgebildet sind, als die aus dem Stand der Technik für die Herstellung eines Sicherungsmittels bekannten Drähte wird gewährleistet, dass die Okklusionswendel 3 hoch flexibel ist und das Sicherungsmittel 9 dennoch eine ausreichende Zugfestigkeit aufweist.

## Patentansprüche

1. Vorrichtung zur Implantation von Okklusionswendeln (3) in Körperhohlräumen oder Blutgefäßen, insbesondere Aneurysmen (12), mit mindestens einer aus einer Vielzahl von Windungen bestehenden, in einem Katheter (1) in Längsrichtung vorschiebbaren Okklusionswendel (3) aus Platin oder einer Platinlegierung und einem das Lumen der Okklusionswendel (3) zumindest teilweise durchlaufenden Sicherungsmittel (9), wobei das Sicherungsmittel (9) in seinen Endbereichen in der Okklusionswendel (3) festgelegt ist, das Sicherungsmittel (9) aus mindestens zwei Drähten besteht und jeder Draht einen Durchmesser von weniger als 0,02 mm aufweist, wobei zumindest ein Teil der Drähte aus einem Metall mit Formgedächtniseigenschaften besteht und **dadurch gekennzeichnet, dass** die Drähte parallel verlaufen.

2. Vorrichtung nach Anspruch 1, **dadurch gekennzeichnet, dass** das Sicherungsmittel (9) aus zwei bis vier Drähten besteht.

3. Vorrichtung nach Anspruch 1 oder 2, **dadurch gekennzeichnet, dass** die Drähte aus einer Nickel-Titan-Legierung, insbesondere aus Nitinol bestehen.

4. Vorrichtung nach einem der Ansprüche 1 bis 3, **dadurch gekennzeichnet, dass** das Sicherungsmittel (9) zu einer übergeordneten Struktur vorgeformt ist, die es bei Ausbringen aus einem zur Platzierung der Okklusionswendel (3) verwendeten Katheter (1) einnimmt.

5. Vorrichtung nach einem der Ansprüche 1 bis 4, **dadurch gekennzeichnet, dass** die Okklusionswendel (3) ein oder mehrere elektrolytisch korrodierbare Stellen (2) aufweist.

6. Vorrichtung nach Anspruch 5, **dadurch gekennzeichnet, dass** die Okklusionswendel (3) mehrere voneinander beabstandete, elektrolytisch korrodierbare Stellen (2) aufweist, wobei in jedem zwischen diesen Stellen (2) gelegenen Segment der Okklusionswendel (3) ein Sicherungsmittel (9) angeordnet ist.

7. Vorrichtung nach einem der Ansprüche 1 bis 4, **gekennzeichnet durch** mehrere voneinander beabstandete Okklusionswendeln (3), wobei zwischen den einzelnen Okklusionswendeln (3) jeweils ein elektrolytisch korrodierbar ausgebildetes Ablöseelement (2) angeordnet ist.

8. Vorrichtung nach Anspruch 7, **dadurch gekennzeichnet, dass** in den einzelnen Okklusionswendeln (3) jeweils ein Sicherungsmittel (9) angeordnet ist.

9. Vorrichtung nach einem der Ansprüche 1 bis 8, **dadurch gekennzeichnet, dass** die Drähte von einer elektrisch isolierenden Umhüllung umgeben sind.

10. Vorrichtung nach einem der Ansprüche 1 bis 9, **dadurch gekennzeichnet, dass** sich das Sicherungsmittel (9) vom proximalen bis zum distalen Ende der Okklusionswendel (3) oder des abtrennbaren Segments (5) der Okklusionswendel (3) erstreckt.

11. Vorrichtung nach einem der Ansprüche 1 bis 10, **dadurch gekennzeichnet, dass** das Sicherungsmittel (9) an einem oder beiden seiner Enden mit der Okklusionswendel (3) direkt verbunden ist.

12. Vorrichtung nach einem der Ansprüche 1 bis 10, **dadurch gekennzeichnet, dass** das Sicherungsmittel (9) an einem oder beiden seiner Enden über Übergangsstücke (10', 10") in der Okklusionswendel (3) festgelegt ist, welche mit dem Sicherungsmittel (9) und der Okklusionswendel (3) verbunden sind.

13. Vorrichtung nach Anspruch 11 oder 12, **dadurch gekennzeichnet, dass** das Sicherungsmittel (9) zumindest in einem Endbereich über eine reibschlüssige Verbindung an der Okklusionswendel (3) oder einem Übergangsstück (10', 10") festgelegt ist.

14. Vorrichtung nach einem der Ansprüche 1 bis 13, **dadurch gekennzeichnet, dass** sich proximal an die Okklusionswendel (3) eine als Führungsdraht (4) ausgebildete Einführhilfe anschließt.

15. Vorrichtung nach einem der Ansprüche 1 bis 14, **dadurch gekennzeichnet, dass** die Vorrichtung in Kombination mit einem Katheter (1) vorliegt, durch den die Okklusionswendel (3) zum zu verschließenden Körperhohlraum oder Blutgefäß vorschiebbar ist.

## Claims

1. Device for the implantation of occlusion helixes (3) into body cavities or blood vessels, in particular aneurysms (12), with at least one occlusion helix (3) consisting of platinum or a platinum alloy, comprising a plurality of windings, and being movably arranged in longitudinal direction within a catheter (1), and one securing means (9) passing at least partially through the lumen of the occlusion helix (3), with said securing means (9) being fixed in its end areas inside the occlusion helix (3), the securing means (9) consists of at least two wires with each of the wires having a diameter of less than 0.02 mm in which at least a part of the wires is made of a metal having shape-memory properties, **characterized in that** the wires extend parallelly.

2. The device according to claim 1, **characterized in that** the securing means (9) consists of two to four wires.

3. Device according to claim 1 or 2, **characterized in that** the wires consist of a nickel-titanium alloy, in particular of nitinol.

4. Device according to any one of the claims 1 to 3, **characterized in that** the securing means (9) has been preformed into a superimposed structure which it assumes when it is released from a catheter (1) used for the placement of the occlusion helix (3).

5. Device according to any one of the claims 1 to 4, **characterized in that** the occlusion helix (3) has been provided with one or several electrolytically corrodible locations (2).

6. The device according to claim 5, **characterized in that** the occlusion helix (3) has several spaced locations (2) that are electrolytically corrodible, and a securing means (9) is arranged between these locations (2) in each segment of the occlusion helix (3).

7. Device according to any one of claims 1 to 4, **characterized by** several spaced occlusion helixes (3), with one electrolytically corrodible severance element (2) each being arranged between the individual occlusion helixes (3).

8. The device according to claim 7, **characterized in that** a securing means (9) each is arranged in the individual occlusion helixes (3).

9. Device according to any one of the claims 1 to 8, **characterized in that** the wires are embraced by an electrically insulating sheathing or coating.

10. Device according to any one of the claims 1 to 9, **characterized in that** the securing means (9) extends from the proximal to the distal end of the occlusion helix (3) or the separable segment (5) of the occlusion helix (3).

11. Device according to any one of the claims 1 to 10, **characterized in that** the securing means (9) is directly attached at one or both of its ends to the occlusion helix (3).

12. Device according to any one of the claims 1 to 10, **characterized in that** the securing means (9) at one or both of its ends is fixed via transition elements (10', 10") inside the occlusion helix (3), said elements being connected with the securing means (9) and the occlusion helix (3).

13. Device according to claim 11 or 12, **characterized in that** the securing means (9) is attached at least in one end area to the occlusion helix (3) or a transition element (10', 10") by means of a frictional connection.

14. Device according to any one of the claims 1 to 13, **characterized in that** an insertion aid in the form of a guide wire (4) is arranged proximally to the occlusion helix (3).

15. Device according to any one of the claims 1 to 14, **characterized in that** the device is provided in combination with a catheter (1) through which the occlusion helix (3) can be moved forward towards the body cavity or blood vessel to be occluded.

**Revendications**

1. Dispositif pour l'implantation de spirales occlusives (3) dans des cavités corporelles ou des vaisseaux sanguins, en particulier des anévrysmes (12), avec au moins une spirale occlusive (3) en platine ou en un alliage de platine composée d'une multiplicité de spires et insérable en direction longitudinale dans un cathéter (1) et avec un moyen de fixation (9) parcourant au moins partiellement le lumen de la spirale occlusive (3), dans lequel le moyen de fixation (9) est attaché dans ses régions d'extrémité dans la spirale occlusive (3), le moyen de fixation (9) se compose d'au moins deux fils et chaque fil présente un diamètre de moins de 0,02 mm, dans lequel au moins une partie des fils se compose d'un métal ayant des propriétés de mémoire de forme et **caractérisé en ce que** les fils sont parallèles.

2. Dispositif selon la revendication 1, **caractérisé en ce que** le moyen de fixation (9) se compose de deux à quatre fils.

3. Dispositif selon la revendication 1 ou 2, **caractérisé en ce que** les fils se composent d'un alliage nickel-titane, en particulier de Nitinol.

4. Dispositif selon l'une quelconque des revendications 1 à 3, **caractérisé en ce que** le moyen de fixation (9) est préformé à une structure pré-imposée, qu'il prend lorsqu'il sort d'un cathéter (1) utilisé pour placer la spirale occlusive (3).

5. Dispositif selon l'une quelconque des revendications 1 à 4, **caractérisé en ce que** la spirale occlusive (3) présente un ou plusieurs endroits (2) pouvant être corrodés par électrolyse.

6. Dispositif selon la revendication 5, **caractérisé en ce que** la spirale occlusive (3) présente plusieurs endroits espacés l'un de l'autre (2) qui peuvent être corrodés par électrolyse, dans lequel un moyen de fixation (9) est disposé dans chaque segment de la spirale occlusive (3) situé entre ces endroits (2).

7. Dispositif selon l'une quelconque des revendications 1 à 4, **caractérisé par** plusieurs spirales occlusives (3) espacées l'une de l'autre, dans lequel un élément dissoluble (2) réalisé de façon corrodable par électrolyse est disposé entre les spirales occlusives individuelles (3).

8. Dispositif selon la revendication 7, **caractérisé en ce qu'**un moyen de fixation (9) est respectivement disposé dans les spirales occlusives individuelles (3).

9. Dispositif selon l'une quelconque des revendications 1 à 8, **caractérisé en ce que** les fils sont entourés d'une gaine électriquement isolante.

10. Dispositif selon l'une quelconque des revendications 1 à 9, **caractérisé en ce que** le moyen de fixation (9) s'étend de l'extrémité proximale jusqu'à l'extrémité distale de la spirale occlusive (3) ou du segment séparable (5) de la spirale occlusive (3).

11. Dispositif selon l'une quelconque des revendications 1 à 10, **caractérisé en ce que** le moyen de fixation (9) est directement relié par une ou ses deux extrémités à la spirale occlusive (3).

12. Dispositif selon l'une quelconque des revendications 1 à 10, **caractérisé en ce que** le moyen de fixation (9) est attaché par une ou ses deux extrémités dans la spirale occlusive (3) au moyen de pièces de transition (10', 10"), qui sont reliées au moyen de fixation (9) et à la spirale occlusive (3).

13. Dispositif selon la revendication 11 ou 12, **caractérisé en ce que** le moyen de fixation (9) est relié au moins dans une région d'extrémité à la spirale occlusive (3) ou à une pièce de transition (10', 10") au moyen d'une liaison par friction.

14. Dispositif selon l'une quelconque des revendications 1 à 13, **caractérisé en ce qu'**une aide à l'introduction réalisée sous forme de fil de guidage (4) se raccorde en position proximale à la spirale occlusive (3).

15. Dispositif selon l'une quelconque des revendications 1 à 14, **caractérisé en ce que** le dispositif se présente en combinaison avec un cathéter (1), à travers lequel la spirale occlusive (3) peut être introduite jusqu'à la cavité corporelle ou jusqu'au vaisseau sanguin à fermer.

Fig. 1

Fig. 2

Fig. 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 9909894 A1
- WO 2004014239 A1
- EP 1582152 A
- WO 2004062509 A
- DE 10010840 A1